# EUROPEAN PATENT APPLICATION

(11) **EP 4 169 439 A1**
(43) Date of publication of application: **26.04.2023**
(21) Application number: 20940685.9
(22) Date of filing: 19.06.2020
(51) Int. Cl.: A61B 5/00, H04L 12/24, H04W 24/04

(54) **WIRELESS MEDICAL DEVICE, CENTRAL MONITORING STATION, AND WIRELESS MEDICAL MONITORING SYSTEM AND METHOD**

(71) Applicant: Shenzhen Mindray Bio-Medical Electronics Co., Ltd., Shenzhen, Guangdong 518057 (CN)
(72) Inventor: CHI, Yunfei, Shenzhen, Guangdong 518057 (CN); DAI, Weiwei, Shenzhen, Guangdong 518057 (CN); LIU, Yan, Shenzhen, Guangdong 518057 (CN); MING, Liqiang, Shenzhen, Guangdong 518057 (CN); XING, Runsen, Shenzhen, Guangdong 518057 (CN); NIU, Yanguo, Shenzhen, Guangdong 518057 (CN)
(74) Representative: KIPA AB
(86) International application number: PCT/CN2020/097187
(87) International publication number: WO 2021/253427

(57) **Abstract**

A wireless medical device (01), a central monitoring station (04), and a wireless medical monitoring system and method. The system comprises a wireless medical device (01), a wireless access point (02), a wireless control unit (03) and a central monitoring station (04), wherein the wireless medical device (01) and the central monitoring station (04) access a wireless network by means of the wireless access point (02) and communicate with each other by means of the wireless network; the wireless medical device (01) collects and processes at least one physiological parameter signal of a patient to obtain physiological data of the patient, and can also monitor network state information of the wireless access point (02) where same is located at the same time to obtain first network state information; the wireless medical device (01) sends the physiological data and the first network state information to the central monitoring station (04) by means of the wireless network; and the central monitoring station (04) at least displays the physiological data and stores the first network state information.

## Description

### TECHNICAL FIELD

The disclosure relates to technical field of medical devices, specifically related to a wireless medical device, a central monitoring station, and a wireless medical monitoring system and method.

### BACKGROUND

With the rapid development of science and technology, mobile medical devices and wearable medical devices are more and more widely used in hospitals. Mobile medical devices and wearable medical devices follow the movement of patients, acquire physiological data of patients in real time, and send the acquired physiological data to a central monitoring station through wireless networks, so that doctors can monitor physical conditions of the patients in real time. However, it is difficult for the wireless network of the hospital to achieve complete coverage of all areas. Generally, the network signal in some areas is poor or there is some interference, which easily makes the mobile medical devices or wearable medical devices entering these areas be disconnected from the network of the central monitoring station. Therefore, it is very important to monitor the wireless network.

### SUMMARY

The embodiments of this disclosure have provided a wireless medical device, a central monitoring station, and a wireless medical monitoring system and method for monitoring a network state of a wireless network.

An embodiment of this disclosure provides a wireless medical monitoring system, which includes a wireless medical device, a wireless access point, a wireless control unit and a central monitoring station; wherein
the wireless access point is configured to provide a wireless connection hot spot;
the wireless control unit is configured to constitute a wireless network with the wireless access point and control the wireless access point;
the wireless medical device and the central monitoring station access the wireless network through the wireless access point, the wireless medical device and the central monitoring station communicate through the wireless network;
the wireless medical device is configured to acquire at least one physiological parameter signal of a patient, process the physiological parameter signal, and obtain physiological data of the patient; wherein the wireless medical device is configured to simultaneously monitor network state information of the wireless access point to obtain first network state information, and transmit the physiological data and the first network state information to the central monitoring station through the wireless network;
the central monitoring station is at least configured to display the physiological data and store the first network state information.

An embodiment of this disclosure provides a wireless medical monitoring system, which includes a monitor, a central monitoring station and at least one wireless medical device; wherein the monitor is in communication connection with the central monitoring station and communicates data with the wireless medical device through a wireless network;
the wireless medical device is configured to acquire at least one physiological parameter signal of a patient, process the physiological parameter signal, and obtain physiological data of the patient; wherein the wireless medical device is configured to simultaneously monitor network state information of the wireless network to obtain first network state information, and transmit the physiological data and the first network state information to the central monitoring station through the monitor;
the central monitoring station is at least configured to display the physiological data and store the first network state information.

An embodiment of this disclosure provides a wireless medical device, which is configured to communicate with a central monitoring station through a wireless network; wherein the wireless medical device includes:
a signal acquisition circuit, which is configured to acquire at least one physiological parameter signal by using a physiological parameter sensor;
a first processor, which is configured to process the physiological parameter signal which is acquired by the signal acquisition circuit to obtain physiological data of a patient, monitor network state information of the wireless network to obtain first network state information, and transmit the physiological data and the first network state information to the central monitoring station through the wireless network; wherein the central monitoring station is at least configured to display the physiological data and store the first network state information.

An embodiment of this disclosure provides a central monitoring station, which includes a communication module, a processor, a memory and a display;
the communication module is configured to communicate with at least one wireless medical device through a wireless network, and the wireless medical device accesses the wireless network through a wireless access point;
the processor is connected with the communication module and configured to obtain physiological data of a patient and first network state information through the communication module, wherein the physiological data is acquired by the wireless medical device, the first network state information is network state information of the wireless access point, and the first network state information is monitored by the wireless medical device;
the memory is configured to store the first network state information;
the display is configured to display the physiological data.

An embodiment of this disclosure provides a wireless medical monitoring method which is applied to a wireless medical device, wherein the wireless medical monitoring method includes:
acquiring at least one physiological parameter signal of a patient;
processing the physiological parameter signal to obtain physiological data of the patient and transmitting the physiological data to a central monitoring station through a wireless network;
monitoring network state information of the wireless network to obtain first network state information, transmitting the first network state information to the central monitoring station through the wireless network, wherein the first network state information is analyzed by the central monitoring station to obtain a wireless network analysis result.

An embodiment of this disclosure provides a wireless medical monitoring method which is applied to a central monitoring station, wherein the wireless medical monitoring method includes:
acquiring physiological data of a patient and first network state information through a wireless network, wherein the physiological data is acquired by a wireless medical device, the wireless medical device accesses the wireless network through a wireless access point, the first network state information is network state information of the wireless access point, and the first network state information is monitored by the wireless medical device;
displaying the physiological data; and
storing the first network state information, wherein the first network state information is analyzed to obtain a wireless network analysis result.

An embodiment of this disclosure provides a computer-readable storage medium, including a program that can be executed by a processor to implement the method described above.

Based on the wireless medical device, central monitoring station, wireless medical monitoring system and method of the above embodiments, in addition to transmitting the physiological data of the patient obtained by itself to the central monitoring station through the wireless network, the wireless medical device can also simultaneously monitor the network state information of the wireless access point which communicates with itself, obtain the first network state information, and transmit the first network state information to the central monitoring station through the wireless network, and the central monitoring station can at least display the physiological data of the patient and store the first network state information, so that the wireless medical device can be configured to monitor the network state of the wireless network in real time.

### BRIEF DESCRIPTION OF THE DRAWINGS

FIG. 1 is a structural diagram of a monitor according to an embodiment of this disclosure.
FIG.2 is a structural diagram of a monitor networking system used in a hospital according to an embodiment of this disclosure.
FIG.3 is a structural diagram of a wireless medical monitoring system according to an embodiment of this disclosure.
FIG.4 shows a display effect of a wireless network analysis interface according to an embodiment of this disclosure.
FIG. 5 is a flow chart of a method for comprehensively analyzing data in network log file and second network state information through a central monitoring station according to an embodiment of this disclosure.
FIG.6 is a structural diagram of another wireless medical monitoring system according to an embodiment of this disclosure.
FIG.7 is a structural diagram of another further wireless medical monitoring system according to an embodiment of this disclosure.
FIG.8 is a structural diagram of a wireless medical device according to an embodiment of this disclosure.
FIG.9 is a flow chart of a wireless medical monitoring method according to an embodiment of this disclosure.
FIG. 10 is a structural diagram of a central monitoring station according to an embodiment of this disclosure.
FIG. 11 is a flow chart of another wireless medical monitoring method according to an embodiment of this disclosure.
FIG. 12 is a flow chart of another further wireless medical monitoring method according to an embodiment of this disclosure.

### DESCRIPTION OF THE PREFERRED EMBODIMENTS

This disclosure will be further described in detail below through specific embodiments in combination with the accompanying drawings. In different embodiments, similar elements adopt associated similar reference numbers. In the following embodiments, many details are described in order to make this disclosure better understood. However, one skilled in the art can easily recognize that some of the features can be omitted in different cases, or can be replaced by other elements, materials and methods. In some cases, some operations related to this disclosure are not shown or described in the description, in order to avoid the core part of this disclosure being inundated by too many descriptions. For one skilled in the art, it is not necessary to describe these related operations in detail, as they can fully understand the relevant operations according to the description in this disclosure and the general technical knowledge in the art.

In addition, the features, operations, or features described in the specification may be combined in any appropriate manner to form various embodiments. At the same time, the steps or actions in the method description can also be exchanged or adjusted in order in a manner obvious to one skilled in the art.

The terms "first", "second", etc. in the specification are operable to distinguish different objects, rather than to describe a specific order. In addition, the terms "connection", and "linkage" mentioned in this disclosure include direct and indirect connection (linkage), unless otherwise specified.

At present, wireless networks in hospitals usually have blind spots. The network signals in these blind spots are poor, or there is some interference. When patients with mobile medical devices and wearable medical devices move to these areas, the network connection between these medical devices and the central monitoring station may be disconnected. At this time, doctors cannot observe the physical condition of the patients in real time through the central monitoring station, which results in a certain threat to the life safety of patients. Therefore, it is necessary to accurately locate the blind spot area in the hospital wireless network through some technical means, so as to optimize and avoid medical accidents, and this has become one of the problems that the hospital urgently needs to solve.

The analysis for the network state of the wireless network can be conducted by using a network analysis tool to analyze the maximum response time, minimum response time, network device availability and other parameters, between the host which loaded with the network analysis tool to a certain network device. However, this operation manner has the following disadvantages. 1), the network report formed by this detection method is only an overview report of overall operation of the entire wireless network, and cannot accurately locate network problems; 2), this detection method cannot accurately locate blind spot area of the wireless network.

Based on this, a scheme of this disclosure is proposed. In the embodiment of this disclosure, a wireless medical device and a central monitoring station access a wireless network through a wireless access point and communicate through the wireless network, the wireless medical device acquires at least one physiological parameter signal of a patient and processes it to obtain physiological data of the patient. At the same time, the wireless medical device also monitors network state information of the wireless access point which it communicates with to obtain first network state information; the wireless medical device transmits the obtained physiological data and the first network state information to the central monitoring station through the wireless network; the central monitoring station at least display the physiological data and store the first network state information.

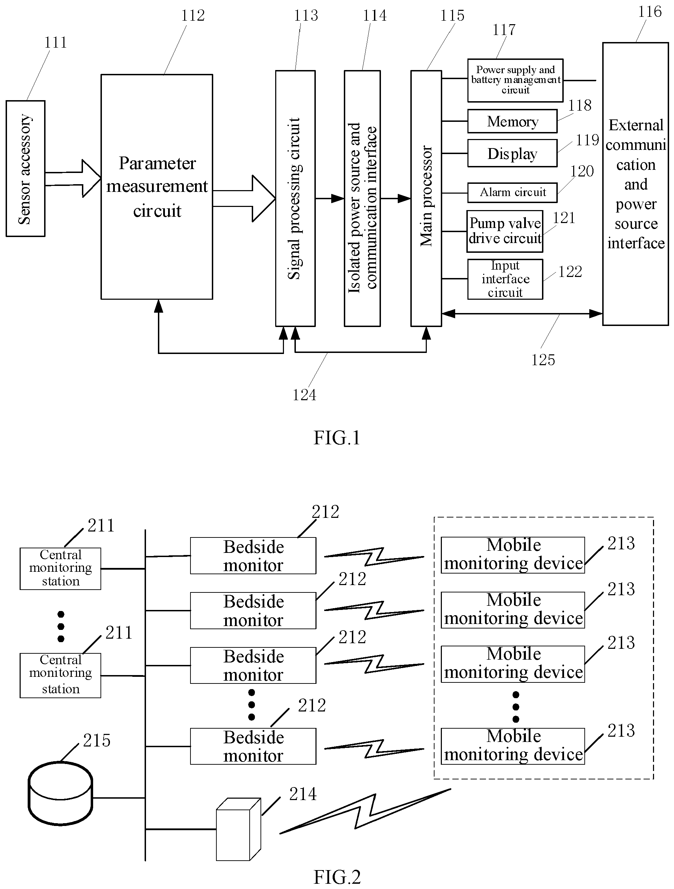

Referring FIG. 1, a structure diagram of a monitor is provided. The monitor can include an independent housing. A housing panel has a sensor interface zone in which multiple sensor interfaces are integrated for connecting with external physiological parameter sensor accessories 111. The housing panel further includes a small IXD (interactive design) display zone, a display 119, an input interface circuit 122, and an alarm circuit 120 (such as an LED alarm zone), etc. The monitor has a parameter processing module which can have an external communication and power source interface 116 for communicating with a main unit and taking power from the main unit. The parameter processing module also supports a build-out parameter module, can form a plug-in monitor main unit by means of inserting the parameter module, can be used as part of the monitor, or can be connected to the main unit via a cable, with the build-out parameter module being used as an external accessory of the monitor.

The internal circuit of the parameter processing module is disposed inside the housing. As shown in FIG. 1 the internal circuit includes a parameter measurement circuit 112, a front-end signal processing circuit 113, and a main processor 115. The parameter measurement circuit 112 may at least includes at least one parameter measurement circuit which is selected from an electrocardiogram parameter measurement circuit, a respiration parameter measurement circuit, a body temperature parameter measurement circuit, a blood oxygen parameter measurement circuit, a non-invasive blood pressure parameter measurement circuit, an invasive blood pressure parameter measurement circuit, etc. The parameter measurement circuit 112 is respectively electrically connected with the sensor accessory 111 which is inserted externally through the corresponding sensor interface, with an output end thereof being coupled to the front-end signal processing circuit 113. A communication port of the front-end signal processing circuit 113 is coupled to the main processor 115, and the main processor 115 is electrically connected to the external communication and power source interface 116. The sensor accessory 111 includes detection accessories corresponding to detection of physiological parameters, such as ECG physiological parameter, respiration physiological parameter, blood oxygen physiological parameter, blood pressure physiological parameter and body temperature physiological parameter and so on. The parameter measurement circuit 112 is mainly configured to connect the sensor accessory 111 to obtain the acquired physiological parameter signal. The parameter measurement circuit 112 and sensor accessory 111 corresponding to various physiological parameters can use common circuits in the prior art. The front-end signal processing circuit 113 completes the sampling and analogy-to-digital conversion of the output signal of the parameter measurement circuit 112, and outputs control signals to control the measurement process of the physiological signals. These parameters include but are not limited to parameters of electrocardiogram, respiration, body temperature, blood oxygen saturation, non-invasive blood pressure, and invasive blood pressure. The front-end signal processing circuit 113 can be realized by a single-chip microcomputer (such as mixed signal single chip microcomputer of LPC2136 from PHLIPS company or ADuC7021 from ADI, and so on) or other semiconductor devices and can also be realized by an ASIC or FPGA. The front-end signal processing circuit 113 can be powered by an isolated power source. The sampled data is simply processed and packaged, and then transmitted to the main processor through the isolated communication interface. For example, the front-end signal processing circuit 113 can be coupled to the main processor 115 through the isolated power source and communication interface 114. The reason that the front-end signal processing circuit 113 is powered by an isolated power source is that the DC/DC power source isolated by a transformer plays a role in isolating the patient from the power supply device, and the main purpose is: 1. isolating the patient, in which the application part is floated above the ground through the isolation transformer such that the leakage current of the patient is small enough; and 2. preventing the voltage or energy in the application of defibrillation or electric scalpel from affecting the boards and devices of the intermediate circuit such as the main control board (guaranteed by creepage distance and electrical clearance). Of course, the front-end signal processing circuit 113 may also be connected to the main processor 115 through a cable 124. The main processor 15 completes the calculation of physiological parameters and transmits the calculation results and waveforms of the parameters to the main unit (such as a main unit with a display, PC, central station, etc.) through the external communication and power source interface 16. The main processor 15 is connected with the external communication and power source interface 116 through a cable 125 for communicating and taking power. The parameter processing module is further provided with a power supply and battery management circuit 117, which takes power from the main unit through the external communication and power interface 116 and supplies the power to the main processor 115 after processing, such as rectification and filtering. The power supply and battery management circuit 117 can also monitor, manage and protect the power obtained from the main unit through the external communication and power interface 116. The external communication and power source interface 116 may be one or a combination of local zone network interfaces composed of Ethernet, Token Ring, Token Bus, and the optical fibre distributed data interface (FDDI) as the backbone of these three networks, may also be one or a combination of wireless interfaces such as infrared, Bluetooth, WIFI, and WMTS communication, or may also be one or a combination of wired data connection interfaces such as RS232 and USB. The external communication and power source interface 116 may also be one or a combination of the wireless data transmission interface and the wired data transmission interface. The main unit can be any computer device, such as the main unit of the monitor, ECG machine, ultrasonic diagnostic instrument and computer. The monitor can be formed by installing the matching software on the main unit. The main unit can also be a communication device, such as a mobile phone. The parameter processing module transmits the data to the mobile phone supporting Bluetooth communication through the Bluetooth interface to realize the remote transmission of data. After the main processor 115 completes the calculation of physiological parameters, it can also determine whether the physiological parameters are abnormal. If yes, it can alarm through the alarm circuit 120. The memory 118 may store the intermediate and final data of the monitor and program instructions or code for execution by the main processor 115 or the like. If the monitor has the function of blood pressure measurement, it may also include a pump valve drive circuit 121 for inflation or deflation under the control of the main processor 115.

As shown in FIG. 2, a monitor networking system used in a hospital is provided. By using the monitor networking system, data of a monitor may be stored as a whole, patient information and nursing information can be managed centrally and stored in association, which facilitates the storage of historical data and associated alarming. In the system shown in FIG. 2, one bedside monitor 212 can be provided for each hospital bed and the bedside monitor 212 can be the above-mentioned multi-parameter monitor or plug-in monitor. In some examples, the bedside monitor 212 can also be paired with one mobile monitoring device 213. The mobile monitoring device 213 provides a simple and portable parameter processing module as it can be worn on the body of a patient for mobile monitoring of the patient. With the wired or wireless communication between the mobile monitoring device 213 and the bedside monitor 212, the monitored physiological data generated during the mobile monitoring can be transmitted to the bedside monitor 212 for displaying or be transmitted to the central monitoring station 211 through the bedside monitor 212 for doctors or nurses to view. In addition, the mobile monitoring device 213 may further directly transmit, through a wireless network node 214 which is arranged in the hospital, the monitored physiological data generated during the mobile monitoring process to the central monitoring station 211 for storage and display, or transmit, through a wireless network node 214 which is arranged inside the hospital, the monitored physiological data generated during the mobile monitoring process to a data server 215 for storage. It can be seen that, the data which is corresponding to the monitored physiological data and is displayed on the bedside monitor 212 may originate from the sensor accessory directly connected to the monitor, or from the mobile monitoring device 213, or from the data server.

Each of the units or modules configured to execute each aforementioned step can be stored in one or more of the aforementioned memories, to implement the aforementioned monitor or monitoring system respectively in the aforementioned embodiments. Each functional module includes each instruction set configured to execute the corresponding steps in the above method. The above module or program (i.e., instruction set) never needs to be limited to discrete software programs, processes or modules. Therefore, in various embodiments, each sub-module of these modules can be combined or rearranged. Therefore, in some embodiments of this disclosure, the memory can store a subset of the modules or data structures described above.

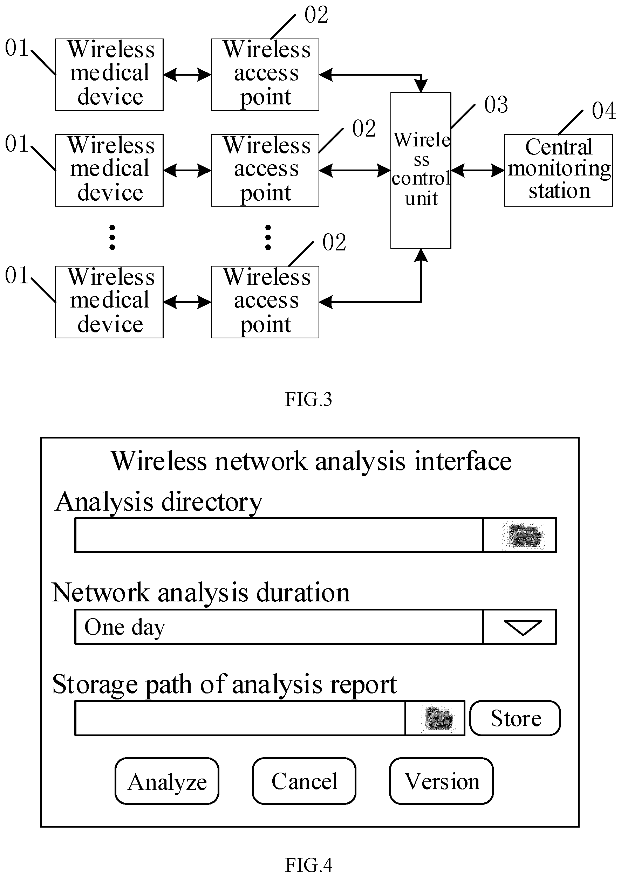

Refer to FIG.3, which is a structural diagram of a wireless medical monitoring system according to an embodiment of this disclosure. The wireless medical monitoring system includes a wireless medical device 01, a wireless access point 02, a wireless control unit 03 and a central monitoring station 04.

The wireless access point 02 is configured to provide a wireless connection hot spot. The wireless control unit 03 is configured to constitute a wireless network with the wireless access point 02 and to control the wireless access point 02. The wireless medical device 01 and the central monitoring station 04 access the wireless network through the wireless access point 02 and communicate through the wireless network. The wireless medical device 01 can be managed and monitored by the central monitoring station 04.

The wireless medical device 01 is configured to acquire at least one physiological parameter signal of a patient (such as ECG physiological parameter signal, respiration physiological parameter signal, blood oxygen physiological parameter signal, blood pressure physiological parameter signal and body temperature physiological parameter signal and so on), process the physiological parameter signal, and obtain physiological data of the patient. The wireless medical device 01 is configured to simultaneously monitor network state information of the wireless access point 02 which communicates with itself to obtain first network state information, and transmit the physiological data and the first network state information which are obtained by the wireless medical device 01 to the central monitoring station 04 through the wireless network. The central monitoring station 04 is at least configured to display the physiological data and store the first network state information.

The first network state information can include one or more of SSID (Service Set Identifier), BSSID (Basic Service Set Identifier) , name, location, channel, channel utilization rate, and RSSI (Received Signal Strength Indicator) value.

In this way, the wireless medical device 01 can not only execute the monitoring process of physiological parameter of the patient, but also simultaneously execute the monitoring process of the wireless network, in which network state information of the wireless access point 02 which communicates with the network status information is monitored. Moreover, the wireless medical device 01 can use the same wireless network to transmit the monitored physiological data of the patient and the monitored first network state information to the central monitoring station 04. The monitoring process of physiological parameter of the patient and the monitoring process of the network share the same wireless network, which realizes the real-time monitoring of the network state information of the wireless network. The central monitoring station 04 can store the first network state information which is monitored by the wireless medical device 01 to further analyze the first network state information and form a network analysis report.

In practical application, the wireless access point 02 and the wireless control unit 03 can be two different devices, or a special device with both wireless access point function and wireless control function.

In one embodiment, after acquiring the first network state information which is monitored by the wireless medical device 01, the central monitoring station 04 is also configured to analyze the first network state information and obtain a wireless network analysis result. In one embodiment, the first network state information can be stored in a network log file. For example, in one embodiment, the wireless medical device 01 can regularly transmit the first network state information to the central monitoring station 04 through the wireless network. The central monitoring station 04 detects whether a value of the first network state information changes. If at least one value of the first network state information changes, the currently received first network state information is recorded in the network log file. In another embodiment, the central monitoring station 04 can record the first network state information in the network log file after receiving the first network state information transmitted by the wireless medical device 01, regardless of whether the first network state information changes.

In one embodiment, when the central monitoring station 04 determines that a network log file exists in an analysis directory, it can analyze data in the network log file to obtain a network event, and then analyze the network event according to a preset rule to obtain a wireless network analysis result. The network event at least includes online and offline events of a wireless medical device, and the wireless network analysis result at least reflects an offline situation of wireless network.

Specifically, the central monitoring station 04 can set at least one analysis directory, which is configured to store and manage a network log file. The central monitoring station 04 can display a wireless network analysis interface which includes a second menu for selecting the analysis directory, detecting an operation of a user on the wireless network analysis interface, and determining the analysis directory according to the operation of the user to the second menu on the wireless network analysis interface. For example, a display effect of the wireless network analysis interface can be seen in FIG.4. The user can click a folder icon in the "Analysis Directory" menu to find a storage path of the analysis directory and select the analysis directory. Specifically, when the wireless network analysis function needs to be executed, the user can start the wireless network analysis tool through the central monitoring station 04. At this time, the central monitoring station 04 can display the wireless network analysis interface at its display interface. The user can select the analysis directory of the network log file through the second menu on the wireless network analysis interface. After confirmation, the central monitoring station 04 determines whether a network log file exists in the selected analysis directory. If yes, it analyzes data in the network log file in the selected analysis directory to get the network event, and then analyzes the network event according to the preset rule to obtain the wireless network analysis result.

When analyzing the network event according to the preset rule, the central monitoring station 04 is specifically configured to count offline situations of the wireless medical device 01 that satisfies a preset condition, based on the network event within a preset network analysis duration.

Among them, the preset network analysis duration can be one day, two days, one week, two weeks and other time periods. The central monitoring station 04 can count the offline situations of the wireless medical device 01 according to the network event within the preset time period. In one embodiment, the user can preset a network analysis duration on the wireless network analysis interface. Specifically, the central monitoring station 04 can display the wireless network analysis interface at its display interface. The wireless network analysis interface includes a first menu for setting the network analysis duration. The central monitoring station 04 detects operation of the user on the wireless network analysis interface and determines the network analysis duration according to a setting operation of the user to the first menu on the wireless network analysis interface. For example, on the wireless network analysis interface as shown in FIG.4, the user can preset the network analysis duration in the "Network Analysis Duration" menu. The user can directly enter the network analysis duration, or select the network analysis duration through a drop-down menu. For example, if the network analysis duration is preset as "one day", the central monitoring station 04 analyzes the data in the network log file of the selected analysis directory to obtain the network events, and then count the offline situations of the wireless medical device 01 that satisfies the preset condition from the network events in the latest day.

Among them, counting the offline situation of wireless medical device 01 that satisfies the preset condition can include at least one of the followings: (1) Counting a total offline duration of the wireless medical device 01 each day. (2) For each preset duration range of each day, counting each offline duration which falls into the preset duration range. For example, take a day as unit, the preset duration range can be fewer than or equal to 15s, greater than 15s but fewer than 60s, and/or greater than or equal to 60s; such that each offline duration of the wireless medical device 01 which is fewer than or equal to 15s in one day is counted, each offline duration of the wireless medical device 01 which is greater than 15s but fewer than 60s in one day is counted, or each offline duration of the wireless medical device 01 which is greater than or equal to 60s in one day is counted. (3) For each day, counting offline frequency of the wireless medical device 01 in each preset period. For example, take a day as unit. For each day, counting offline frequency of the wireless medical device 01 in each hour, or in each two hours, and so on. (4) For each day, counting the offline frequency of the wireless medical device 01 in which a channel utilization rate of the wireless medical device 01 falls into a channel utilization rate threshold range. For example, take a day as unit. For each day, counting offline frequency of the wireless medical device 01 in which the channel utilization rate of the wireless medical device 01 falls into the channel utilization rate threshold range which is greater than 60%, between 40% and 60%, or less than 40%. (5) For each day, counting information of the wireless access point which communicates with the wireless medical device 01 when the wireless medical device 01 is offline.

In one embodiment, after the central monitoring station 04 obtains the wireless network analysis result, it can record the wireless network analysis result in a file so that user can view and analyze the wireless network analysis result.

In one embodiment, after receiving the wireless network analysis result, the central monitoring station 04 is also configured to generate a graphical report based on the wireless network analysis result. In this way, the user without network related knowledge can also understand the analysis result, so as to further optimize the wireless network. Specifically, the graphical report can include at least one of the followings: an internal and external distribution diagram of offline duration threshold of the wireless medical device 01, a frequency distribution diagram of wireless access point which communicates with the wireless medical device when the wireless medical device is offline, a regional distribution diagram of offline time of the wireless medical device, a utilization rate distribution diagram of channel(s) which is used by the wireless medical device when the wireless medical device is offline, an offline frequency distribution diagram of the wireless medical device, and an offline frequency distribution diagram of the wireless medical device in 24 hours.

Specifically, for the internal and external distribution diagram of offline duration threshold, the central monitoring station 04 can analyze the first network state information to obtain the offline situation of the wireless medical device 01, determine whether each offline duration falls within the offline duration threshold, count each offline duration falling within the offline duration threshold and each offline duration falling outside the offline duration threshold, and then obtain the internal and external distribution diagram of offline duration threshold according to the counted results. The internal and external distribution diagram of offline duration threshold can be a distribution graph of each day which is obtained by taking a day as unit. For example, the central monitoring station 04 can analyze the network event according to the preset condition (2) which belongs to the offline situation of wireless medical device 01 that satisfies the preset condition, as discussed before, to obtain each offline duration falling within the offline duration threshold (which is greater than 15s but fewer than 60s) and each offline duration which is fewer than 15s or greater than 60s (that is, obtaining each offline duration falling outside the offline duration threshold), such that the internal and external distribution diagram of offline duration threshold of the wireless medical device 01 at that day can be obtained.

The central monitoring station 04 can analyze the first network state information to count information of the wireless access point which communicates with the wireless medical device 01 when the wireless medical device 01 is offline each time. For example, it can perform statistical analysis on a daily basis to obtain the frequency distribution diagram of wireless access point which communicates with the wireless medical device when the wireless medical device is offline. The user can view the offline situation of each wireless access point through the distribution diagram, and analyze failure of each wireless access point.

The central monitoring station 04 can analyze the first network state information, count each offline time of the wireless medical device 01, and obtain the regional distribution diagram of offline time of the wireless medical device 01. The user can further analyze and determine offline reason for the wireless medical device 01 according to the regional distribution diagram of offline time.

The central monitoring station 04 can analyze the first network state information and count offline frequency of the wireless medical device 01 when the channel utilization rate falls within the channel utilization rate threshold, such as the offline frequency of the wireless medical device 01 in which the channel utilization rate of the wireless medical device 01 falls into the channel utilization rate threshold range which is greater than 60%, between 40% and 60%, or less than 40%, so as to obtain the utilization rate distribution diagram of channel(s) which is used by the wireless medical device when the wireless medical device is offline. The idle and busy state of the wireless network when the wireless medical device 01 is offline can be known through the distribution diagram.

The central monitoring station 04 can analyze the first network state information, count offline frequency of the wireless medical device 01 in each preset period, such as offline frequency in each two hours, offline frequency in each day, etc., so as to obtain the offline frequency distribution diagram of the wireless medical device 01, according to which the failure of the wireless medical device 01 can be analyzed. The offline frequency distribution diagram of the wireless medical device in 24 hours, can also be obtained, and according to which the movement of the patient with the wireless medical device 01 can be analyzed.

In one embodiment, after generating the graphical report, the central monitoring station 04 can also store the graphical report. The storage path of the graphical report can be preset, or be set by the user.

Specifically, the central monitoring station 04 can display the wireless network analysis interface at its display interface. The wireless network analysis interface can include a storage menu for storing the graphical report. The central monitoring station 04 detects operation of the user on the wireless network analysis interface, and stores the graphical report according to the selected storage path based on the operation of the user to the storage menu on the wireless network analysis interface. For example, on the wireless network analysis interface as shown in FIG.4, the user can set the storage path of the graphical report in the "Analysis Report Storage Path" menu, and then click the "Storage" button. At this time, the central monitoring station 04 stores the graphical report according to the set storage path.

In one embodiment, as shown in FIG.4, the wireless network analysis interface can also include "Analysis" function key and/or "Version" function key. When the "Analysis" function key is triggered, the central monitoring station 04 starts the function of analyzing the data in the network log file, enabling the user to manually start the wireless network analysis function provided in this disclosure. Of course, the central monitoring station 04 can also automatically start the wireless network analysis function. For example, the central monitoring station 04 can automatically conduct wireless network analysis after receiving the first network state information. The user can know version, copyright and other information of the current network analysis tool (running in the central monitoring station 04 and providing the wireless network analysis function of this disclosure) by triggering the "Version" function key.

In one embodiment, the central monitoring station 04 can also realize a mailbox function. After generating the graphical report, the graphical report can be transmitted to the user through the mailbox. For example, the central monitoring station 04 can provide the user with mailbox setting function, allowing the user to set email address for receiving reports. After generating the graphical report, the central monitoring station 04 can transmit the graphical report to the user according to the email address. For example, it can transmit graphical report immediately after generating it, or regularly.

In one embodiment, the central monitoring station 04 can also regularly acquire network state information of the wireless access point 02 to obtain second network state information. At this time, the data in the network log file and the second network state information can be comprehensively analyzed to obtain the wireless network analysis result. The second network state information can include one or more of MAC (Media Access Control) address, BSSID, IP (Internet Protocol) address, name, location, number, channel, channel utilization rate and interference information.

Specifically, the central monitoring station 04 determines whether the second network state information acquired by the central monitoring station 04 exists in the analysis directory. If so, it analyzes the second network state information, obtains a mapping relationship between BSSID and name of the wireless access point 02, and caches the same. When determining that a network log file exists in the analysis directory, it analyzes the data in the network log file by integrating the mapping relationship. If the second network state information acquired by the central monitoring station 04 does not exist in the analysis directory, it analyze the data in the network log file when determining that a network log file exists in the analysis directory.

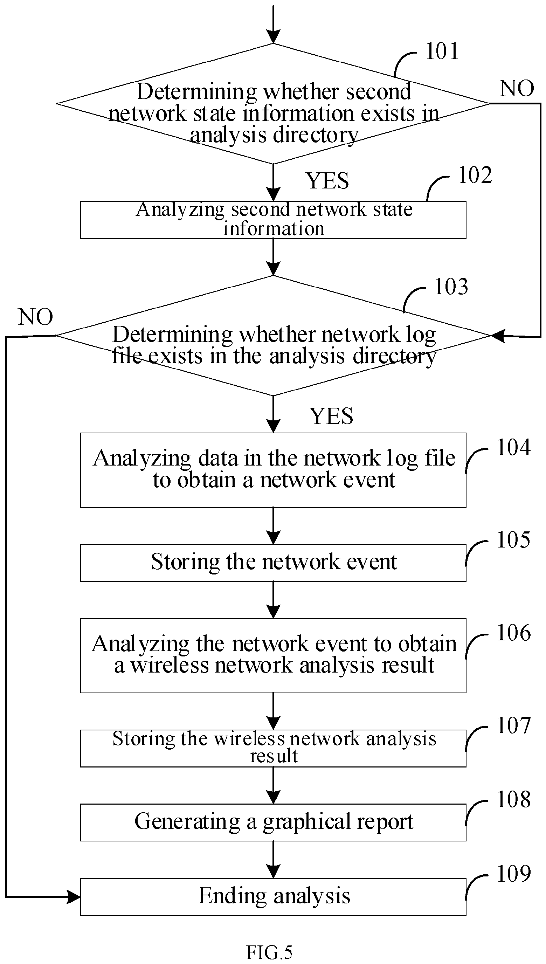

Based on this, refer to FIG.5, which is a flow chart of a method for comprehensively analyzing data in network log file and second network state information through the central monitoring station 04 according to an embodiment of this disclosure. The method can include the following steps.

In step 101, whether second network state information exists in an analysis directory, is determined.

After the central monitoring station 04 starts the wireless network analysis function, it firstly determine whether the second network state information acquired by the central monitoring station 04 exists in the analysis directory. If yes, step 102 is executed; otherwise, step 103 is executed.

In step 102, the second network state information is analyzed.

When the central monitoring station 04 determines that the second network state information exists in the analysis directory, it analyzes the second network state information, extracts the mapping relationship between BSSID and name of the wireless access point 02 from the second network state information, and caches the same. The BSSID is configured to distinguish a network channel of the wireless network. For one wireless access point 02, it can correspond to multiple BSSIDs, and each BSSID can connect with one wireless medical device 01. That is, one wireless access point 02 can connect to multiple wireless medical devices 01. By analyzing the second network state information and extracting the mapping relationship between the BSSID and the name of wireless access point 02 from the second network state information, the name of wireless access point 02 which communicates with the wireless medical device 01 can be accurately obtained.

In step 103, whether a network log file exists in the analysis directory is determined.

The central monitoring station 04 determines whether a network log file recorded by the central monitoring station 04 exists in the analysis directory. The network log file is configured to store the first network state information which is monitored by the wireless medical device 01. If yes, step 104 is executed, otherwise step 109 is executed.

In step 104, data in the network log file is analyzed to obtain a network event.

When the central monitoring station 04 determines that the second network state information does not exist, but a network log file exists in the analysis directory, it analyzes the data in the network log file to obtain a network event, which at least includes online and offline events of the wireless medical device 01.

When the central monitoring station 04 determines that the second network state information exists in the analysis directory and a network log file exists, it analyzes the data in the network log file by integrating the mapping relationship between the BSSID and the name of wireless access point 02 to obtain the network event, which at least includes the online and offline events of wireless medical device 01. By extracting the mapping relationship between the BSSID and the name of the wireless access point 02 from the second network state information, the name of the wireless access point 02 which communicates with the wireless medical device 01 can be accurately known, so that the first network state information can be complementary. After analyzing the data in the network log file, the wireless access point 02 corresponding to the obtained network event can be accurately known, and the accurate mapping relationship between the network event and wireless access point 02 can be obtained.

In step 105, the network event is stored.

After acquiring the network event, the central monitoring station 04 can store the network event of each wireless medical device 01 to a file which belongs to each wireless medical device 01, so that the user can view and analyze the wireless network operation situation of each wireless medical device 01.

In step 106, the network event is analyzed to obtain a wireless network analysis result.

After the central monitoring station 04 obtains the network event, it analyzes the network event according to a preset rule to obtain the wireless network analysis result, which at least reflects an offline situation of the wireless network. Or, when receiving the instruction that the user needs to view and analyze the wireless network operation of the wireless medical device 01, the network event is read from the corresponding file which stores the same, and then the network event is analyzed according to the preset rule to obtain the wireless network analysis result. Specifically, it is possible to analyze the network event within a preset network analysis duration and count the offline situations of the wireless medical device 01 that satisfies a preset condition.

After acquiring the wireless network analysis result, the central monitoring station 04 performs following steps 107 and 108.

In step 107, the wireless network analysis result is stored.

After receiving the wireless network analysis result, the central monitoring station 04 can record the wireless network analysis result in a file for storage, so that the user can view and analyze the wireless network analysis result.

In step 108, a graphical report is generated.

After the central monitoring station 04 obtains the wireless network analysis result, it can generate a graphical report based on the wireless network analysis result. The graphical report can include, for example, an internal and external distribution diagram of offline duration threshold of the wireless medical device 01, a frequency distribution diagram of wireless access point which communicates with the wireless medical device when the wireless medical device is offline, a regional distribution diagram of offline time of the wireless medical device, a utilization rate distribution diagram of channel(s) which is used by the wireless medical device when the wireless medical device is offline, an offline frequency distribution diagram of the wireless medical device, and an offline frequency distribution diagram of the wireless medical device in 24 hours. In this way, users without network related knowledge can also understand the analysis result, so as to further optimize the wireless network.

In step 109, the analysis is ended.

The wireless medical device, central monitoring station, wireless medical monitoring system provided by an embodiment of this disclosure, communicate through the wireless network. The wireless medical device can use the same one wireless network to simultaneously execute the monitoring process of physiological parameter of the patient and the monitoring process of the network, can monitor the network state information of the wireless access point which communicates with itself through the monitoring process of the network to obtain the first network state information, transmit the first network state information to the central monitoring station, which can store the first network state information and can also further analyze the first network state information to obtain the wireless network analysis result. Through the wireless network analysis result, the offline situation of the wireless access point which communicates with the wireless medical device can be known at least, so as to optimize the wireless network. The central monitoring station can also form a graphical report based on the wireless network analysis result, so that user without network related knowledge can understand the analysis result, so as to further optimize the wireless network. On the other hand, the central monitoring station can also provide user with a simple and friendly user interaction interface, such as the wireless network analysis interface shown in FIG.4, through which user can manually start the wireless network analysis function, set the network analysis duration, select the analysis directory of network log file, and set the storage path of the graphical report.

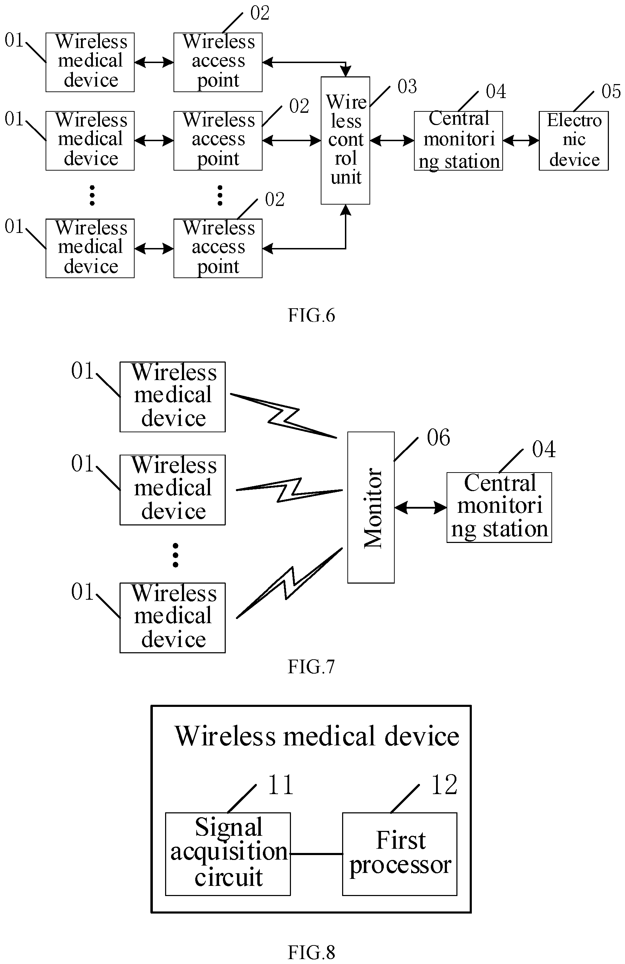

Based on the concept of this disclosure, as shown in FIG.6, a structural diagram of another wireless medical monitoring system provided for an embodiment of this disclosure is provided. The wireless medical monitoring system includes at least one wireless medical device 01, a wireless access point 02, a wireless control unit 03, a central monitoring station 04, and an electronic device 05 that communicates with the central monitoring station 04. The electronic device 05, for example, can be a desktop computer, laptop, tablet, PDA (Personal Digital Assistant), digital TV and other devices.

Wherein, the wireless access point 02 is configured to provide a wireless connection hotspot, the wireless control unit 03 is configured to constitute a wireless network with the wireless access point 02 and control the wireless access point 02. The wireless medical device 01 and the central monitoring station 04 access the wireless network through the wireless access point 02, the wireless medical device 01 and the central monitoring station 04 communicate through the wireless network, and the wireless medical device 01 can be managed and monitored by the central monitoring station 04.

The wireless medical device 01 can realize the functions of the wireless medical device 01 in FIG.1. The central monitoring station 04 is at least configured to display the physiological data and store the first network state information which is monitored by the wireless medical device 01.

The electronic device 05 is configured to obtain the first network state information from the central monitoring station 04, analyze the first network state information, and obtain the wireless network analysis result. The specific method for the electronic device 05 to analyze the first network state information can refer to the process of the central monitoring station 04 for analyzing the first network state information in the above embodiments. That is, in this embodiment, the central monitoring station 04 is configured to display the physiological data of the patient and store the first network state information which is monitored by the wireless medical device 01, while the wireless network analysis function performed by the central monitoring station 04 is performed by the electronic device 05.

Based on this, the wireless network analysis function provided in this disclosure can be deployed to the central monitoring station or any electronic device. When the wireless network analysis function is deployed to any electronic device, the first network state information which is monitored by the wireless medical device can be exported from the central monitoring station to the electronic device deployed with the wireless network analysis function. The wireless network analysis function can be run at the electronic device to analyze the first network state information, obtain the wireless network analysis result, and form a graphical report. The scheme of this disclosure is characterized by flexible deployment and convenient use, which can accurately locate the problems existing in the wireless network and guide the user to optimize the wireless network.

Based on the concept of this disclosure, as shown in FIG.7, a structural diagram of another further wireless medical monitoring system is provided by an embodiment of this disclosure. The wireless medical monitoring system includes a monitor 06, a central monitoring station 04 and at least one wireless medical device 01. The monitor 06 communicates with the central monitoring station 04 and transmits data to the wireless medical device 01 through the wireless network.

The wireless medical device 01 is configured to acquire at least one physiological parameter signal of a patient, process the physiological parameter signal, and obtain the physiological data of the patient. The wireless medical device 01 is configured to simultaneously monitor network state information of the wireless network which communicates with itself to obtain first network state information, and transmit the physiological data and the first network state information to the central monitoring station 04 through the monitor 06. The central monitoring station 04 is at least configured to display the physiological data transmitted by the wireless medical device 01 and store the first network state information transmitted by the wireless medical device 01. The central monitoring station 04 can also realize the functions of the central monitoring station 04 in the above embodiments.

In the wireless medical monitoring system shown in FIG.7, the monitor 06 can be, for example, the monitor described in FIG.1 or FIG.2. The monitor 06 can be used as a wireless access point to provide wireless network data transmission for the wireless medical device 01. The wireless data transmission between the wireless medical device 01 and central monitoring station 04 can be realized with the help of existing monitors in the hospital. The system is simple to build.

Based on the concept of this disclosure, as shown in FIG.8, a structural diagram of a wireless medical device is provided for an embodiment of this disclosure. The wireless medical device is configured to communicate with a central monitoring station through a wireless network. The wireless medical device includes a signal acquisition circuit 11 and a first processor 12.

Wherein, the signal acquisition circuit 11 is configured to acquire at least one physiological parameter signal by using a physiological parameter sensor. The first processor 12 is configured to process the physiological parameter signal acquired by the signal acquisition circuit 11 to obtain physiological data of the patient. The first processor 12 is also configured to monitor network state information of the wireless network which communicates with the wireless medical device to obtain first network state information, and transmit the obtained physiological data and first network state information to the central monitoring station through the wireless network. The central monitoring station is configured to at least display the physiological data and store the first network state information.

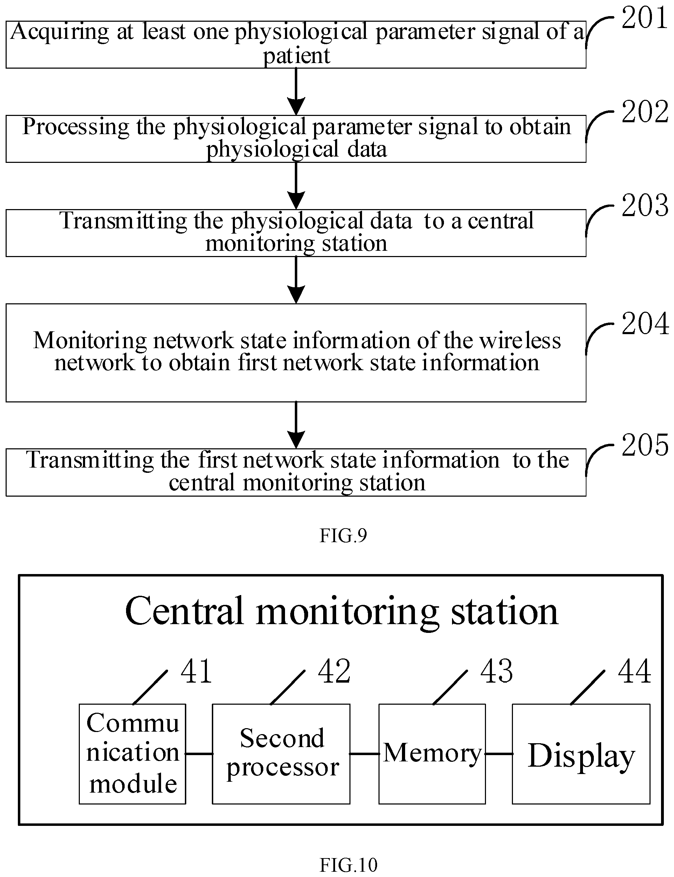

Referring to FIG.9, a flow chart of a wireless medical monitoring method according to an embodiment of this disclosure can be applied to the wireless medical device 01 or the wireless medical device of the embodiment corresponding to FIG.8. The method can include the following steps.

In step 201, at least one physiological parameter signal of a patient is acquired.

The wireless medical device can perform a monitoring process of physiological parameter of the patient, and can use a physiological parameter sensor to acquire at least one physiological parameter signal of the patient, such as body temperature signal, blood pressure signal, ECG signal, respiratory signal, blood oxygen signal, etc.

In step 202, the physiological parameter signal is processed to obtain physiological data.

After the wireless medical device acquires at least one physiological parameter signal of the patient, it processes the physiological parameter signal to obtain the physiological data of the patient, such as, physiological data of body temperature, blood pressure, ECG, respiration, blood oxygen and others.

In step 203, the physiological data is transmitted to a central monitoring station.

After the wireless medical device obtains the physiological data of the patient, it transmits the physiological data to the central monitoring station through the wireless network. For example, the wireless medical device and central monitoring station can access the same wireless network through a wireless access point, and they can communicate through the wireless network. After the wireless medical device obtains the physiological data of the patient, it can transmit the physiological data of the patient to the central monitoring station through the wireless network.

In step 204, network state information of the wireless network is monitored to obtain first network state information.

The wireless medical device and the central monitoring station can access the same wireless network through the wireless access point. The wireless medical device can not only execute the monitoring process of physiological parameter of the patient, but also execute the monitoring process of the wireless network, monitor the network state information of the wireless network, and obtain the first network state information. The first network state information includes one or more of SSID, BSSID, name, location, channel, channel utilization rate, and RSSI value.

In step 205, the first network state information is transmitted to the central monitoring station.

After the wireless medical device obtains the first network state information, it transmits the first network state information to the central monitoring station through the wireless network. Wherein the first network state information is analyzed by the central monitoring station to obtain a wireless network analysis result.

The wireless medical monitoring method provided in this embodiment can be applied to a wireless medical device. It can not only execute the monitoring process of physiological parameter of patient, but also simultaneously execute the monitoring process of the wireless network, monitor the network state information of the wireless access point which communicates with the wireless medical device, and use the same wireless network to transmit the physiological data of the patient and the monitored first network state information to the central monitoring station. The monitoring process of physiological parameter of patient and the monitoring process of the wireless network share the same wireless network, which realizes the real-time monitoring of the network state information of the wireless network. After the first network state information is transmitted to the central monitoring station, the central monitoring station can store the first network state information, so as to further analyze the first network state information, obtain the wireless network analysis result, and form a network analysis report to guide the user to optimize the wireless network.

Based on the concept of this disclosure, as shown in FIG.10, a structural diagram of a central monitoring station according to an embodiment of this disclosure is disclosed. The central monitoring station includes a communication module 41, a second processor 42, a memory 43 and a display 44.

The communication module 41 is configured to communicate with at least one wireless medical device through a wireless network, and the wireless medical device accesses the wireless network through a wireless access point.

The second processor 42 is connected with the communication module 41 and is configured to acquire physiological data of a patient and first network state information through the communication module 41. The physiological data is acquired by the wireless medical device, and the first network state information is network state information of the wireless access point which communicates with the wireless medical device. The first network state information is monitored by the wireless medical device. In practical applications, the second processor 42 can receive a second type of data which is actively transmitted by the wireless medical device, actively acquire the second type of data from the wireless medical device after the wireless medical device has established a connection with the central monitoring station. The wireless medical device can be, for example, the wireless medical device of the corresponding embodiment of FIG.8.

The memory 43 is configured to store the first network state information acquired by the second processor 42.

The display 44 is configured to display the physiological data acquired by the second processor 42.

In one embodiment, the second processor 42 is also configured to analyze the acquired first network state information to obtain a wireless network analysis result.

In one embodiment, the second processor 42 is also configured to acquire the network state information of the wireless access point regularly through the communication module 41 to obtain the second network state information. The second network information is used for comprehensive analysis with the first network state information to obtain the wireless network analysis result.

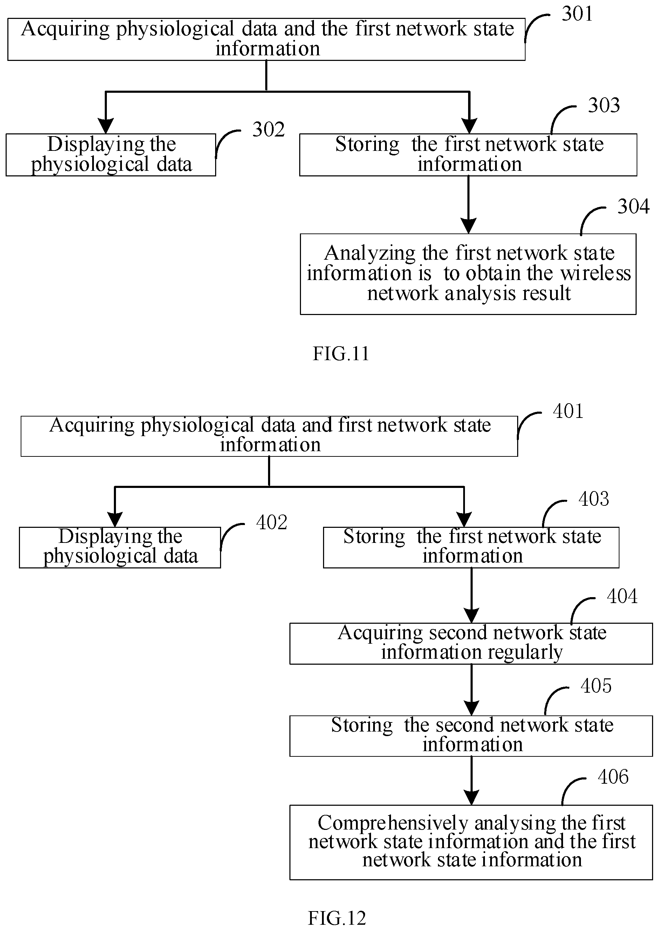

Referring to FIG. 11, a flow chart of another further wireless medical monitoring method provided for an embodiment of this disclosure is disclosure. The wireless medical monitoring method can be applied to the central monitoring station 04 or the central monitoring station in the corresponding embodiment of FIG. 10. The method can include the following steps.

In step 301, physiological data and first network state information are acquired.

The central monitoring station acquires the physiological data of the patient and the first network state information through a wireless network. The physiological data is acquired by the wireless medical device. The wireless medical device accesses the wireless network through the wireless access point, and the central monitoring station also accesses the wireless network. The central monitoring station can communicate with the wireless medical device through the wireless network. The first network state information is network state information of the wireless access point which communicates with the wireless medical device, and the first network state information is monitored by the wireless medical device.

In step 302, the physiological data is displayed.

The central monitoring station can display the physiological data of the patient to the user after acquiring the physiological data of the patient through the wireless network.

In step 303, the first network state information is stored.

After the central monitoring station obtains the first network state information through the wireless network, it stores the first network state information, and analyzes the same to obtain a wireless network analysis result.

After the central monitoring station obtains the first network state information through the wireless network or stores the first network state information, it can also perform the following step 304.

In step 304, the first network state information is analyzed to obtain the wireless network analysis result.

After the central monitoring station obtains the first network state information or stores the first network state information, it analyzes the first network state information to obtain the wireless network analysis result, so as to accurately locate problems in the wireless network and guide user to optimize the wireless network.

The wireless medical monitoring method provided in this embodiment can be applied to the central monitoring station. It can obtain the physiological data of the patient transmitted by the wireless medical device and the first network state information of the wireless access point which communicates with the wireless medical device, then display the physiological data, and at least store the first network state information, so that the wireless network analysis result can be obtained after analyzing the first network state information. For example, the central monitoring station can analyze the first network state information to obtain the wireless network analysis result. The wireless network analysis result can accurately locate the problem in the wireless network, and then guide user to optimize the wireless network.

Based on the corresponding embodiment of FIG. 11, refer to FIG. 12, which is a flow chart of another wireless medical monitoring method according to an embodiment of this disclosure that can be applied to the above-mentioned central monitoring station 04 or the central monitoring station of the corresponding embodiment of FIG. 10. The method can include the following steps.

In step 401: physiological data of a patient and first network state information are acquired through a wireless network.

In step 402, the physiological data is displayed.

In step 403, the first network state information is stored.

The specific process from step 401 to step 403 corresponds to step 301 to step 303, and is not repeated here.

In step 404, second network state information is acquired regularly.

The central monitoring station not only acquires the physiological data of the patient and the first network state information through the wireless network, but also regularly acquires the network state information of the wireless access point which communicates with the wireless medical device through the wireless network to obtain the second network state information. The second network state information includes one or more of MAC address, BSSID, IP address, name, location, number, channel, channel utilization rate and interference information.

In step 405, the second network state information is stored

After acquiring the second network state information, the central monitoring station stores the second network state information. The second network state information can be comprehensive analyzed with the first network state information to obtain a wireless network analysis result.

After the central monitoring station acquires the second network state information and stored it, it can also perform the following step 406.

In step 406, the first network state information and the second network state information are comprehensively analyzed.

After acquiring and storing the second network state information, the central monitoring station can comprehensively analyze the first network state information and the second network state information to obtain the wireless network analysis result. For example, the first network state information and the second network state information can be comprehensively analyzed using the method corresponding to the embodiment in FIG.5 to obtain the wireless network analysis result.

The wireless medical monitoring method provided in this embodiment can be applied to the central monitoring station. It can obtain physiological data and the first network state information from the wireless medical device through the wireless network. At the same time, it can regularly acquire the network state information of the wireless access point which communicates with the wireless medical device through the same wireless network to obtain the second network state information. Then the acquired first network state information and second network state information can be comprehensively analyzed to obtain the wireless network analysis result. The wireless network analysis result can accurately locate the problem existing in the wireless network, and then guide user to optimize the wireless network. Moreover, the first network state information can be complemented by the second network state information. After analyzing the data in the network log file, the wireless access point corresponding to each network event can be accurately known, and the accurate mapping relationship between network events and wireless access point can be obtained.

The description has been made with reference to various exemplary embodiments herein. However, those skilled in the art recognizes that changes and modifications can be made to the exemplary embodiments without departing from the scope herein. For example, various operation steps and components for performing operation steps may be implemented in different ways according to a specific application or considering any number of cost functions associated with the operation of the system (for example, one or more steps may be deleted, modified, or incorporated into other steps).

In addition, as understood by those skilled in the art, the principles herein may be reflected in a computer program product on a computer-readable storage medium that is pre-installed with computer-readable program codes. Any tangible, non-transitory computer-readable storage medium can be used, including magnetic storage devices (hard disks, floppy disks, etc.), optical storage devices (CD-ROM, DVD, Blu Ray disks, etc.), flash memory, and/or the like. These computer program instructions can be loaded onto a general-purpose computer, a dedicated computer, or other programmable data processing device to form a machine, so that these instructions executed on a computer or other programmable data processing apparatus can generate an apparatus that implements a specified function. These computer program instructions can also be stored in a computer-readable memory that can instruct a computer or other programmable data processing device to operate in a specific manner, so that the instructions stored in the computer-readable memory can form a manufactured product, including an implementation apparatus that implements a specified function. The computer program instructions can also be loaded onto a computer or other programmable data processing device, so that a series of operating steps are performed on the computer or other programmable device to produce a computer-implemented process, so that the instructions executed on a computer or other programmable data processing device can provide steps for implementing specified functions.

Although the principles herein have been shown in various embodiments, many modifications of structures, arrangements, proportions, elements, materials and components particularly suitable for the specific environmental and operational requirements may be used without departing from the principles and scope of the present disclosure. The above modifications and other changes or amendments will be included in the scope of this disclosure.

The foregoing specific description has been described with reference to various embodiments. However, those skilled in the art will recognize that various modifications and changes can be made without departing from the scope of the present disclosure. Therefore, consideration of the present disclosure will be in an illustrative rather than a restrictive sense, and all such modifications will be included within the scope thereof. Also, the advantages of various embodiments, other advantages, and the solutions to problems have been described above. However, the benefits, advantages, solutions to problems, and any elements that can produce these, or solutions that make them more explicit, should not be interpreted as critical, necessary, or essential. The term "comprising", and any other variants thereof used herein are non-exclusive, so that the process, method, document, or device that includes a list of elements includes not only these elements, but also other elements that are not explicitly listed or do not belong to the process, method, system, document, or device. Furthermore, the term "coupling" and any other variations thereof used herein refer to physical connection, electrical connection, magnetic connection, optical connection, communicational connection, functional connection, and/or any other connection.

It should be noted that a person of ordinary skill in the art could also make several variations and improvements without departing from the concept of this disclosure, and these variations and improvements would all fall within the scope of protection of this disclosure. Therefore, the protection scope of this disclosure should be in accordance with the appended claims.

## Claims

1. A wireless medical monitoring system, **characterized in that**, comprising a wireless medical device, a wireless access point, a wireless control unit and a central monitoring station; wherein
the wireless access point is configured to provide a wireless connection hot spot;
the wireless control unit is configured to constitute a wireless network with the wireless access point and control the wireless access point;
the wireless medical device and the central monitoring station access the wireless network through the wireless access point, wherein the wireless medical device and the central monitoring station communicate through the wireless network;
the wireless medical device is configured to acquire at least one physiological parameter signal of a patient, process the physiological parameter signal, and obtain physiological data of the patient; wherein the wireless medical device is further configured to simultaneously monitor network state information of the wireless access point to obtain first network state information, and transmit the physiological data and the first network state information to the central monitoring station through the wireless network;
the central monitoring station is at least configured to display the physiological data and store the first network state information.

2. The wireless medical monitoring system according to claim 1, **characterized in that**, the central monitoring station is further configured to analyze the first network state information to obtain a wireless network analysis result.

3. The wireless medical monitoring system according to claim 2, **characterized in that**, the first network state information is stored in a network log file.

4. The wireless medical monitoring system according to claim 3, **characterized in that**, the central monitoring station is further configured to analyze data in the network log file to obtain a network event and analyze the network event according to a preset rule to obtain the wireless network analysis result, when determining that the network log file exists in an analysis directory.

5. The wireless medical monitoring system according to claim 4, **characterized in that**, the network event at least comprises online and offline events of the wireless medical device, and the wireless network analysis result at least reflects an offline situation of the wireless network.

6. The wireless medical monitoring system according to claim 5, **characterized in that**, in order to analyze the network event according to a preset rule, the central monitoring station is further configured to count offline situation(s) of the wireless medical device that satisfies a preset condition based on the network event within a preset network analysis duration.

7. The wireless medical monitoring system according to claim 6, **characterized in that**, the central monitoring station is further configured to display a wireless network analysis interface which comprises a first menu for setting the network analysis duration, detect an operation of a user on the wireless network analysis interface and determine the network analysis duration according to a setting operation of the user to the first menu on the wireless network analysis interface.

8. The wireless medical monitoring system according to claim 6, **characterized in that**, the network analysis duration is measured in days;
in order to count offline situation(s) of the wireless medical device that satisfies a preset condition, the central monitoring station is further configured to at least:
count a total offline duration of the wireless medical device each day; for each preset duration range of each day, count each offline duration which falls into the preset duration range; for each day, count offline frequency of the wireless medical device in each preset period; for each day, count offline frequency of the wireless medical device in which a channel utilization rate of the wireless medical device falls into a channel utilization rate threshold range; for each day, count information of the wireless access point when the wireless medical device is offline.

9. The wireless medical monitoring system according to any one of claims 2-8, **characterized in that**, the central monitoring station is further configured to generate a graphical report based on the wireless network analysis result.

10. The wireless medical monitoring system according to claim 9, **characterized in that**, the graphical report comprises at least one of followings:
an internal and external distribution diagram of offline duration threshold of the wireless medical device, a frequency distribution diagram of the wireless access point when the wireless medical device is offline, a regional distribution diagram of offline time of the wireless medical device, a utilization rate distribution diagram of channel(s) which is used by the wireless medical device when the wireless medical device is offline, an offline frequency distribution diagram of the wireless medical device, and an offline frequency distribution diagram of the wireless medical device in 24 hours.

11. The wireless medical monitoring system according to claim 9, **characterized in that**, the central monitoring station is further configured to display a wireless network analysis interface which comprises a storage menu for storing the graphical report, to detect an operation of a user on the wireless network analysis interface and to store the graphical report along a selected storage path according to the operation of the user to the storage menu on the wireless network analysis interface.

12. The wireless medical monitoring system according to claim 9, **characterized in that**, the central monitoring station is further configured to transmit the graphical report to a user by email.

13. The wireless medical monitoring system according to claim 4, **characterized in that**, the central monitoring station is further configured to display a wireless network analysis interface which comprises a second menu for selecting the analysis directory, to detect an operation of a user on the wireless network analysis interface, and to determine the analysis directory according to the operation of the user to the second menu on the wireless network analysis interface.

14. The wireless medical monitoring system according to claim 2, **characterized in that**, the central monitoring station is further configured to regularly acquire the network state information of the wireless access point to obtain second network state information, to comprehensively analyze data in a network log file and the second network state information to obtain the wireless network analysis result.

15. The wireless medical monitoring system according to claim 14, **characterized in that**, the second network state information comprises one or more of: MAC address, BSSID, IP address, name, location, number, channel, channel utilization rate and interference information.

16. The wireless medical monitoring system according to claim 15, **characterized in that**, the central monitoring station is further configured to determine whether the second network state information which is acquired by the central monitoring station exists in an analysis directory;
if the second network state information which is acquired by the central monitoring station exists in the analysis directory, the central monitoring station is further configured to analyze the second network state information, obtain and buffer a mapping relationship between BSSID and name of the wireless access point, and analyze the data in the network log file by integrating the mapping relationship when determining that the network log file exists in the analysis directory;
if the second network state information which is acquired by the central monitoring station does not exist in the analysis directory, the central monitoring station is further configured to analyze the data in the network log file when determining that the network log file exists in the analysis directory.

17. The wireless medical monitoring system according to claim 1, **characterized in that**, the first network state information comprises one or more of: SSID, BSSID, name, location, channel, channel utilization rate, and RSSI value.

18. The wireless medical monitoring system according to claim 1, **characterized in that**, the wireless medical monitoring system further comprises an electronic device that communicates with the central monitoring station;
wherein the electronic device is configured to acquire the first network state information from the central monitoring station, analyze the first network state information and obtain a wireless network analysis result.

19. A wireless medical monitoring system, **characterized in that**, comprising a monitor, a central monitoring station and at least one wireless medical device; wherein the monitor is in communication connection with the central monitoring station and communicates data with the wireless medical device through a wireless network;
the wireless medical device is configured to acquire at least one physiological parameter signal of a patient, process the physiological parameter signal, and obtain physiological data of the patient; wherein the wireless medical device is configured to simultaneously monitor network state information of the wireless network to obtain first network state information, and transmit the physiological data and the first network state information to the central monitoring station through the monitor;
the central monitoring station is at least configured to display the physiological data and store the first network state information.

20. A wireless medical device which is configured to communicate with a central monitoring station through a wireless network; **characterized in that**, the wireless medical device comprises:
a signal acquisition circuit, which is configured to acquire at least one physiological parameter signal by using a physiological parameter sensor; and
a first processor, which is configured to process the physiological parameter signal which is acquired by the signal acquisition circuit to obtain physiological data of a patient, monitor network state information of the wireless network to obtain first network state information, and transmit the physiological data and the first network state information to the central monitoring station through the wireless network;
wherein the central monitoring station is at least configured to display the physiological data and store the first network state information.

21. A central monitoring station, **characterized in that**, comprising a communication module, a second processor, a memory and a display;
the communication module is configured to communicate with at least one wireless medical device through a wireless network, and the wireless medical device accesses the wireless network through a wireless access point;
the second processor is connected with the communication module and configured to obtain physiological data of a patient and first network state information through the communication module, wherein the physiological data is acquired by the wireless medical device, the first network state information is network state information of the wireless access point, and the first network state information is monitored by the wireless medical device;
the memory is configured to store the first network state information;
the display is configured to display the physiological data.

22. The central monitoring station according to claim 21, **characterized in that**, the second processor is further configured to analyze the first network state information to obtain a wireless network analysis result.

23. The central monitoring station according to claim 21, **characterized in that**, the second processor is further configured to regularly acquire the network state information of the wireless access point to obtain second network state information through the communication module, wherein the second network state information is comprehensively analyzed with the first network state information to obtain the wireless network analysis result.

24. A wireless medical monitoring method which is applied to a wireless medical device, **characterized in that**, the wireless medical monitoring method comprises:
acquiring at least one physiological parameter signal of a patient;
processing the physiological parameter signal to obtain physiological data of the patient and transmitting the physiological data to a central monitoring station through a wireless network;
monitoring network state information of the wireless network to obtain first network state information, transmitting the first network state information to the central monitoring station through the wireless network, wherein the first network state information is analyzed by the central monitoring station to obtain a wireless network analysis result.

25. A wireless medical monitoring method which is applied to a central monitoring station, **characterized in that**, the wireless medical monitoring method comprises:
acquiring physiological data of a patient and first network state information through a wireless network, wherein the physiological data is acquired by a wireless medical device, the wireless medical device accesses the wireless network through a wireless access point, the first network state information is network state information of the wireless access point, and the first network state information is monitored by the wireless medical device;
displaying the physiological data; and
storing the first network state information, wherein the first network state information is analyzed to obtain a wireless network analysis result.

26. The wireless medical monitoring method according to claim 25, **characterized in that**, the wireless medical monitoring method further comprises:
analyzing the first network state information to obtain the wireless network analysis result.

27. The wireless medical monitoring method according to claim 25, **characterized in that**, the wireless medical monitoring method further comprises:
acquiring the network state information of the wireless access point regularly to obtain second network state information and storing the second network state information, wherein the second network state information is comprehensively analyzed with the first network state information to obtain the wireless network analysis result.

28. The wireless medical monitoring method according to claim 27, **characterized in that**, the wireless medical monitoring method further comprises:
comprehensively analyzing the first network state information and the first network state information to obtain the wireless network analysis result.

29. A computer readable storage medium, **characterized in that**, comprising a program that can be executed by a processor to implement the method according to any one of claims 24-28.
